# EUROPÄISCHE PATENTSCHRIFT

(11) **EP 2 029 690 B1**
(45) Veröffentlichungstag und Bekanntmachung des Hinweises auf die Patenterteilung: **25.09.2013**
(21) Anmeldenummer: 07725390.4
(22) Anmeldetag: 21.05.2007
(51) Int. Cl.: C09K 3/32, C04B 20/10, C04B 28/02, C04B 40/00, C09D 5/34, C11D 11/00, C11D 17/06, A61K 8/81, A61K 8/92, A61Q 19/00

(54) **VERFAHREN ZUR HERSTELLUNG VON MIT PFLANZENÖLEN UND PFLANZENÖLDERIVATEN MODIFIZIERTEN BINDEMITTELN UND GERÜSTSTOFFEN SOWIE VERWENDUNG DER HERGESTELLTEN MODIFIZIERTEN BINDEMITTEL UND GERÜSTSTOFFE.**
METHOD FOR PREPARING BINDING AGENTS AND BUILDERS MODIFIED WITH VEGETABLE OILS AND VEGETABLE OIL DERIVATIVES AND USE OF THE PREPARED MODIFIED BINDERS AND BUILDERS.
MÉTHODE DE PRÉPARATION DE LIANTS MODIFIÉS À L'AIDE D'HUILES VÉGÉTALES ET DE DÉRIVÉS D'HUILES VÉGÉTALES ET ADJUVANTS ET UTILISATION DES LIANTS MODIFIÉS ET ADJUVANTS PRÉPARÉS.

(30) Priorität: 24.05.2006 CH 867062006
(43) Veröffentlichungstag der Anmeldung: 04.03.2009
(73) Patentinhaber: Weirich, Harald, 66802 Überherrn-Berus (DE)
(72) Erfinder: Weirich, Harald, 66802 Überherrn-Berus (DE)
(74) Vertreter: Hrovat, Andrea Darinka
(86) Internationale Anmeldenummer: PCT/EP2007/004482
(87) Internationale Veröffentlichungsnummer: WO 2007/134822

(56) Entgegenhaltungen:
- DE-A1- 1 937 460
- DE-A1- 3 812 594
- DE-A1- 4 119 193
- US-A- 3 899 455

## Beschreibung

Die Erfindung betrifft ein Verfahren zur Herstellung von mit Pflanzenölen, Pflanzenölbestandteilen oder Pflanzenölderivaten modifizierten Bindemitteln und Gerüststoffen sowie bevorzugte Verwendungen der erfindungsgemäß hergestellten modifizierten Bindemittel und Gerüststoffe.

Ausgangspunkt für die vorliegende Erfindung bildeten Erkenntnisse, welche sich an eine Erfindung anschließen, die auf denselben Anmelder zurückgeht und als DE-B-41 19 193 veröffentlicht worden ist. Die DE-B-41 19 193 offenbart ein Öl bindendes Mittel auf der Basis eines Polystyrolschaumes bzw. -hartschaumes, bei welchem der Polystyrolschaum zerkleinert, z.B. zerspant, vorliegt und mit einem Pflanzenöl belegt worden ist. Das Pflanzenöl benetzt die Oberfläche der zerspanten Polystyrol-Schaumteilchen und verbessert dadurch deren Eigenschaften ganz erheblich. Grundgedanke war dabei die Bereitstellung eines umweltfreundlichen Produkts aus einem nachwachsenden Rohstoff, vorzugsweise sogar einem Abfallprodukt bei der Pflanzenölherstellung.

Das so erhaltene Bindemittel zeigt eine außergewöhnliche Affinität gegenüber weiterem Öl, gleich welcher Art, und wurde deshalb als Mittel zum Binden von Öl nach Unfällen auf dem Wasser oder dem Land, zum Reinigen von Wasser, zum Reinigen von Behältern oder zum Auffangen von Lecköl vorgeschlagen. Das weitere Öl wird dabei von dem durch die Pflanzenölbelegung modifizierten Polystyrol-Hartschaum wie von einem Löschblatt aufgesogen.

Bedingt durch die Belegung des Polystyrol-Hartschaums mit dem Pflanzenöl ergab sich außerdem eine erstaunlich verbesserte Handhabbarkeit des Bindemittels in Form einer deutlich höheren Schüttdichte und einer erheblich verbesserten Rieselfähigkeit.

Labor- und Demonstrationsversuche des Anmelders des vorgenannten Standes der Technik haben überdies gezeigt, daß bei entsprechender Beladung der Polystyrol-Schaumteilchen mit dem Pflanzenöl und bei dem Einsatz zum Binden von Erdöl bei der innerhalb kürzester Zeit erfolgenden Aufnahme des Erdöls gleichmäßige schwarze Kugeln mit einem Durchmesser von ca. 2 - 4 cm entstanden, die eine erstaunliche Stabilität gegenüber Druck aufwiesen, so daß sie das von ihnen aufgenommene Erdöl auch bei deutlicher Druckeinwirkung nicht wieder freigaben.

Erste Erkenntnisse über die Eignung als Bindemittel auch über den bis dahin vorgesehenen Verwendungszweck als Ölbindemittel hinaus ergaben sich schon augenscheinlich daraus, daß die sich erstaunlicherweise bei einem bestimmten Belegungsgrad des Polystyrolschaumes mit dem Pflanzenöl beim Aufnehmen des Erdöls bildenden schwarzen Kugeln eine leichte Klebrigkeit aufwiesen. Das Phänomen der Bildung der genannten schwarzen Kugeln deutete auf das Vorhandensein bzw. Entstehen starker Ordnungskräfte hin. Um diese zu verstehen, waren Überlegungen aus einem völlig anderen technischen Bereich hilfreich.

Im Zusammenhang mit der Phosphatierung von Metallen ist ein Phänomen bekannt, das sich bei der Verwendung von Schmierstoffen auf Seifenbasis in Verbindung mit Phosphatschichten ergibt. Werden Zink-Phosphatschichten auf einem Werkstück mit Alkaliseifen umgesetzt, entsteht dabei Zinkseife, die zum einen besonders wirksam ist und zum anderen einen geordneten Aufbau auf der Phosphatschicht aufweist. Es scheinen dabei besondere Ordnungskräfte zu wirken, die technisch nutzbar gemacht werden (siehe Dr. Rausch, "Die Phosphatierung von Metallen", Eugen G. Leuze Verlag, Saulgau/Württ., zweite Auflage 1988).

Es war davon auszugehen, daß vergleichbare Ordnungskräfte auch für das Phänomen der starken Bindungswirkung von mit Pflanzenöl belegtem zerkleinertem oder zerspantem Kunststoffschaum, wie dem Polystyrol-(Hart-)Schaum verantwortlich sind. Mit dieser einmal gewonnenen Erkenntnis lag es außerdem auf der Hand, daß die besonderen, bei der Erdölbindung zutage getretenen Eigenschaften des mit dem Pflanzenöl belegten Kunststoffs einer Reihe weiterer interessanter Anwendungen zugeführt werden können.

Davon ausgehend lag der vorliegenden Erfindung daher die Aufgabe zugrunde, den für die Erdölbindung bekanntgewordenen, mit Pflanzenöl belegten Kunststoffschaum weiteren Anwendungen zuzuführen.

Gelöst wird diese Aufgabe durch ein Verfahren zur Herstellung eines modifizierten Bindemittels oder Gerüststoffs auf der Basis eines Kunststoffs wobei das Bindemittel oder der Gerüststoff als Adsorptionsverbindung aus dem zerkleinerten Kunststoff und zumindest einem Pflanzenöl, Pflanzenölbestandteil oder Derivat davon erhältlich ist, gegebenenfalls mit weiteren Bestandteilen oder Zusatzstoffen, wobei der Kunststoff Polystyrol oder ein modifiziertes Polystyrol, ist.

Dabei ist der Begriff der Zerkleinerung weit zu fassen. Die Teilchengröße hängt unter anderem von der Art des eingesetzten Kunststoffmaterials und dem gewünschten Verwendungszweck ab sowie von dem eingesetzten Zerkleinerungsverfahren. Hier können schneidende oder reißende Verfahren eingesetzt werden; wobei der Kunststoff dann zerspant oder zerbürstet wird, und es kann genauso ein Granulat mit einer je nach Verwendungszweck vorherzubestimmender Korngröße als Ausgangsmaterial dienen. Der Begriff des zerkleinerten Materials umfaßt im Rahmen der vorliegenden Erfindung auch ein Granulat beliebiger Korngröße.

Bei dem Einsatz von zerspantem oder zerbürstetem Kunststoffmaterial haben sich Teilchengrößen kleiner als ca. 3 mm, bevorzugt etwa in einem Bereich von 1,5 bis 0,5 mm als sinnvoll erwiesen.

Wenn ein Granulat eingesetzt wird, richtet sich die Korngröße nach der Art des gewählten Herstellungsverfahrens und dem Einsatzzweck in der einem Fachmann an sich geläufigen Weise.

Als Kunststoff kann Polystyrol in Form eines Homo- oder Copolymerisats eingesetzt werden. Polystyrol hat den Verteil, herstellungsbedingt preisgünstig und andererseits in bezug auf die Copolymerisate in Form einer breiten Produktpalette verfügbar zu sein. Gleichwertig oder für bestimmte Anwendungen bevorzugt kann auch ein modifiziertes Polystyrol Verwendung finden. Modifizierte Polystyrole sind vielfältig bekannt. Beispielhaft ist α-Methylstyrol zu nennen.

Für bestimmte Anwendungsformen ist ein Polystyrol-Copolymerisat als Kunststoffmaterial zu bevorzugen, das z. B. ausgewählt ist aus einem Styrol/(Meth-) Acrylat-Copolymer. Dieses Styrol/(Meth-)Acrylat-Copolymer, aber auch ein Homopolymerisat bzw. ein weiterer Kunststoff kann in seiner zerkleinerten Form ebenso als Dispersion eingesetzt werden.

Das für die Bildung der Adsorptionsverbindung und damit des erfindungsgemäßen Bindemittels oder Gerüststoffs dienende Pflanzenöl ist vorzugsweise ausgewählt aus Rapsöl (auch Rüböl oder Rüpsenöl genannt), Sonnenblumenkemöl, Sojaöl, Rizinusöl, Olivenöl, Leinöl, Kokosöl, Palmöl, einem Bestandteil oder Derivat und Mischungen davon. Es soll aber an dieser Stelle darauf hingewiesen werden, daß es sich dabei um eine exemplarische Aufzählung der gängigen Pflanzenöle handelt, und daß grundsätzlich jegliches Pflanzenöl für die Zwecke der vorliegenden Erfindung eingesetzt werden kann.

Als Derivat des Pflanzenöls sind besonders die Ester und Glycerinester der mehrfach ungesättigten Fettsäuren hervorzuheben.

Es ist hinlänglich bekannt, daß die natürlichen pflanzlichen Öle Glycerinester der höheren geradzahligen Fettsäuren, d. h. der Glyceride darstellen und bis zu ca. 97% aus Triglyceriden, bis zu ca. 3% aus Diglyceriden und bis ca. 1% aus Monoglyceriden aufgebaut sind. Tri-, Di- und Monoglyceride bestehen jeweils aus einem Molekül Glycerin, verestert mit 3 Molekülen, 2 Molekülen bzw. 1 Molekül Fettsäure. Die chemischen, physikalischen und biologischen Eigenschaften der Pflanzenöle werden von der Art der Fettsäurekomponente und ihrer Verteilung über die Triglyceridmoleküle bestimmt. Es ist außerdem hinlänglich bekannt, daß der Schmelzpunkt im allgemeinen mit zunehmendem Anteil an langkettigen Fettsäuren bzw. mit abnehmendem Anteil an kurzkettigen oder ungesättigten Fettsäuren steigt. Die Eigenschaften eines Triglycerids werden auch durch die Stellung der verschiedenen Fettsäuregruppen innerhalb des Triglyceridmoleküls bestimmt.

Die Fettsäuren selbst sind überwiegend geradzahlige, geradkettige, aliphatische Monocarbonsäuren mit Kettenlängen von C₄ bis C₂₄. Der Schmelzpunkt einer Fettsäure fällt mit abnehmender Kettenlänge und zunehmender Anzahl von Doppelbindungen. Pflanzenöle sind bei Zimmertemperatur flüssig wegen ihres erheblichen Anteils an ungesättigten Fettsäuren.

Wenn das jeweilige Pflanzenöl als Ester der jeweiligen mehrfach ungesättigten Fettsäuren eingesetzt wird, sind grundsätzlich die Methylester von besonderem Interesse. Hier sind insbesondere Rapsfettsäuremethylester, Rizinolsäuremethylester, Ölsäuremethylester, Linolsäuremethylester, Laurinsäuremethylester zu nennen, wobei diese Aufzählung lediglich exemplarisch ist. Die Möglichkeiten sind mannigfaltig und die Auswahl sowie die gegebenenfalls weitere Umsetzung des pflanzlichen Öls oder seiner Bestandteile mit weiteren Fettsäuren ist für den Fachmann aufgrund seines allgemeinen Fachwissens ohne weiteres möglich.

Die eingangs gestellte Aufgabe wird auch gelöst durch die Verwendung des erfindungsgemäß hergestellten Bindemittels bzw. Gerüststoffs in Baustoffen, insbesondere als Klebstoff oder Dichtungsmasse. Dabei sind Klebstoffe und Dichtungsmassen jeglicher Art gemeint und mitumfaßt.

Ausgehend von den zuvor im Hinblick auf die Bildung der kugelförmigen Gebilde bei der Bindung des Erdöls beobachteten klebrigen Eigenschaften wurden erfolgreich verschiedene Versuche durchgeführt, welche sogar eine herausragende Eignung des erfindungsgemäßen Bindemittels als Klebstoff und Dichtungsmasse bestätigten.

Grundsätzlich bestehen Klebstoffe in ihrer einfachsten Zusammensetzung aus einem Bindemittel, dem noch ein Weichmacher zur Eigenschaftsverbesserung zugesetzt wird. Obwohl solche Weichmacher für die Anwendbarkeit der Klebstoffe als erforderlich angesehen werden, sind doch aus dem Stand der Technik verschiedene Nachteile bekannt, welche mit dem Einsatz solcher Weichmacher einhergehen. Einer der wesentlichen Nachteile ist z.B. eine verringerte Haftungseigenschaft. Aus dieser resultiert der bei den herkömmlichen Klebern bekannte Hinweis, ihn nur auf sauberen, trockenen, fett- und ölfreien Oberflächen zu verwenden.

Es ist ebenso seit langem bekannt und üblich, als Grundlage der Klebstoffe Bindemittel auf der Basis der Homo- oder Copolymere des Styrols oder ein modifiziertes Polystyrol einzusetzen.

In einer bevorzugten Verwendung wird erfindungsgemäß als Kunststoff ein modifiziertes Polystyrol, bevorzugt α-Methylstyrol, eingesetzt.

Zur Herstellung der erfindungsgemäßen Kleber und Dichtungsmassen wurde gemäß einer weiteren bevorzugten Verwendung auf eine vielfältige Palette von Styrol/(Meth)acrylat-Copolymeren zurückgegriffen, welche von der BASF im Handel erhältlich sind. Als wesentlichstes und bekanntestes Produkt ist hierbei Acronal verschiedenster Spezifikation, wie Acronal 290D, Acronal S360D usw. zu nennen. Weitere Vertreter der von der BASF erhältlichen Styrol-Copolymerisate sind beispielsweise Neocryl A 621 als Copolymer von Styrol mit Acrylsäureester, Pliotec LS1 als Terpolymer aus Styrol, Butylacrylat und Methacrylsäure, Rhodopas SB 012 als Copolymer von Styrol mit Butadien oder Synthomer VL 10286 als Terpolymer von Styrol mit Butadien und Acrylnitril.

In bezug auf die Pflanzenölkomponente, die erfindungsgemäß als Weichmacher dient, ist es bevorzugt, weniger das Öl selbst als ein Derivat hiervon einzusetzen. Hier sind insbesondere die Fettsäuren und besonders deren Ester, ganz besonders deren Methylester, wie ausgehend von Rapsöl Rapsfettsäuremethylester (auch kurz RME genannt), Ölsäuremethylester und Linolsäuremethylester, hervorzuheben. Die Auswahl des oder der jeweiligen Methylester für die Zwecke der vorliegenden Verwendung hängt unter anderem von wirtschaftlichen Erwägungen ab, d.h. welche(r) Methylester kostengünstig zur Verfügung stehen.

Der Anteil der Pflanzenölkomponente in dem erfindungsgemäßen Klebstoff oder der Dichtungsmasse liegt bevorzugt bei etwa 5 - 20%, besonders bevorzugt bei etwa 7 - 15%.

Dem Klebstoff oder der Dichtungsmasse können des weiteren ansonsten übliche und als solches bekannte Zusätze, wie beispielsweise Pigmente, zugegeben werden, ohne seine Wirkung nachteilig zu beeinflussen.

Die bevorzugte Verwendung des erfindungsgemäß hergestellten Bindemittels bzw. Gerüststoffs betrifft den Bausektor, d. h. spezifische Produktverbesserungen der im Bausektor eingesetzten Bindemittel, wie hydraulische Bindemittel, Trockenmörtelprodukte, Gips und (nichthydraulischer) Kalk.

Unter hydraulischen Bindemitteln werden solche Bindemittel verstanden, die sowohl an der Luft als auch in Wasser erhärten und Zement, hydraulischen Kalk, Putz- und Mauerbinder umfassen.

Langwierige Versuche haben hier eine Reihe von erstaunlichen Produktverbesserungen ergeben, zu denen eine verbesserte Form- und Bearbeitbarkeit, eine Steigerung der Elastizität, Erhöhung der Zug-, Druck-, Biegewechsel- und Torsionsfestigkeit, eine geringere Feuchtigkeitsaufnahme, Erhöhung der Langlebigkeit und nicht zuletzt eine heute besonders beachtete und damit vorteilhafte umweltgerechte Entsorgung bedingt durch die biologische Abbaubarkeit gehören.

Die mit dem erfindungsgemäßen Bindemittel bzw. Gerüststoff hergestellten Produkte zeichnen sich außerdem durch ein geringes Schüttgewicht und eine erhöhte Rieselfähigkeit aus. Sie zeigen eine drastisch verminderte Staubentwicklung und eine Reduktion der elektrostatischen Aufladung.

Die sich stellende Aufgabe wird gelöst durch ein Verfahren zur Herstellung eines modifizierten Bindemittels oder Gerüststoffs auf der Basis eines Kunststoffs Polystyrol oder modifiziertes Polystyrol, mit zumindest einem Pflanzenöl, Pflanzenölbestandteil oder Pflanzenölderivat, bei dem in einem ersten Verfahrensschritt der Kunststoff in zerspanter oder feinverteilter Form mit dem Pflanzenöl, Pflanzenölbestandteil oder Pflanzenölderivat reagieren gelassen und anschließend in einem zweiten Verfahrensschritt der so modifizierte Kunststoff mit demselben oder einem weiteren Pflanzenöl, Pflanzenölbestandteil oder Pflanzenölderivat reagieren gelassen wird.

Mit diesem erfindungsgemäßen Verfahren hat der Anmelder die bereits in seiner ersten Anmeldung dargelegte, aber noch auf ein Öl bindendes Mittel bezogene Erkenntnis weiterentwickelt, wonach im Anschluß an den ersten Verfahrensschritt, d. h. dort die Belegung des Kunststoffs mit dem Pflanzenöl, nur noch "Öl zu Öl kommt", wodurch die besonderen Produkteigenschaften des bekannten Öl bindenden Mittels, aber auch die besonderen Produkteigenschaften des durch das hier vorgestellte erfindungsgemäße Verfahren gewonnenen modifizierten Bindemittels in seinen verschiedenen Verwendungsmöglichkeiten begründet sind.

Das erfindungsgemäße Verfahren unterscheidet sich von der Herstellung des bekannten Öl bindenden Mittels im wesentlichen dadurch, daß es dort für die Herstellung des Mittels lediglich des ersten Verfahrensschrittes bedurfte, d. h. der Kunststoff, wie Polystyrol, wurde mit dem Pflanzenöl belegt und die dabei bereits gewonnenen besonderen und modifizierten Eigenschaften des Polystyrols ergaben das einsatzbereite Ölbindemittel. Der zweite, hier erfindungsgemäß vorgeschlagene Verfahrensschritt, das weitere Belegen mit Öl, geschah bei dem bekannten Öl bindenden Mittel erst bei seiner Verwendung, d.h. bei seinem Einsatz in der Praxis. Entsprechend war es auch Erdöl oder eine Ölverschmutzung, die an das gemäß dem ersten Verfahrensschritt modifizierte Polystyrol gebunden wurde und nicht, wie hier erfindungsgemäß vorgesehen, ein weiteres Pflanzenöl, dessen Bestandteil oder Derivat. Das erfindungsgemäß hier vorgestellte Bindemittel bzw. der Gerüststoff auf der Basis von Polystyrol als bevorzugt zu verwendendem Kunststoff ist erst nach Durchführen auch des zweiten Verfahrensschritt fertig und für vielfältige Verwendungen einsetzbar.

Aufgrund der Unterschiede zwischen der Herstellung des bekannten Öl bindenden Mittels und des erfindungsgemäßen Verfahrens zur Herstellung eines Bindemittels war es auch erforderlich, daß weniger ein z. B. durch Zerspanen zerkleinertes Polystyrol als vielmehr ein feinverteiltes Polystyrol bzw. ein feinverteilter Kunststoff oder eine Polystyrol- bzw. Kunststoffdispersion zum Einsatz kommt. Auch können die Verwendungsmöglichkeiten des Bindemittels dadurch erweitert werden, daß Polystyrol nicht allein, sondern z. B. in Form von Copolymerisaten des Polystyrols mit weiteren Polymeren eingesetzt wird. Als solche Copolymerisate sind insbesondere solche auf der Basis von Styrol/Acrylat zu nennen. Vorzugsweise werden das feinverteilte Polystyrol oder die Polystyrol-Copolymerisate in Form von Dispersionen eingesetzt.

Die Erfindung betrifft genauso die Verwendung des erfindungsgemäßen Bindemittels oder Gerüststoffs sowie des erfindungsgemäßen Verfahrens zu dessen Herstellung im Bereich der Wasch- und Reinigungsmittel.

Der mit dem Pflanzenöl verbundene Kunststoff kann sich dann, wenn weiteres Öl hinzukommt, zu im wesentlichen gleichmäßigen kugelförmigen Strukturen zusammenballen, die eine hohe Stabilität aufweisen.

Durch die Verbindung des Kunststoffs mit dem Pflanzenöl wird eine echte chemische Verbindung geschaffen und eine besonders hohe Schüttdichte sowie eine besonders gute Rieselfähigkeit des erzielten Produkts wird erreicht.

Im folgenden soll die Erfindung anhand von Ausführungsbeispielen und den beigefügten Abbildungen näher erläutert werden.

Es zeigen:
- Fig. 1a:: eine lichtmikroskopische Aufnahme eines mit Rapsöl belegten Polystyrol-Granulats in zehnfacher Vergrößerung,
- Fig. 1b:: eine lichtmikroskopische Aufnahme, wie in Fig. 1a, jedoch in 57-facher Vergrößerung,
- Fig. 2a:: rasterelektronenmikroskopische Aufnahme eines Polystyrol-Granulat-Partikels, mit Rapsöl belegt, in 50-facher Vergrößerung,
- Fig. 2b:: eine Aufnahme nach Fig. 2a, jedoch in 200-facher Vergrößerung,
- Fig. 3:: kugelförmiges Gebilde aus Polystyrol-Hartschaum, nach der Aufnahme von Erdöl, Angabe des Durchmessers jeweils in mm,
- Fig. 4a:: eine lichtmikroskopische Aufnahme eines mit Erdöl belegten Polystyrol-Granulats in 32-facher Vergrößerung,
- Fig. 4b:: eine Abbildung nach Fig. 4a, jedoch in 57-facher Vergrößerung,
- Fig. 5a:: eine weitere lichtmikroskopische Aufnahme eines mit Erdöl belegten Polystyrol-Granulats in 32-facher Vergrößerung,
- Fig. 5b:: eine Aufnahme nach Fig. 5a, jedoch in 57-facher Vergrößerung,
- Fig. 6a:: eine rasterelektronenmikroskopische Aufnahme eines Querschnitts eines mit Erdöl belegten Polystyrol-Granulat-Agglomerats in 50-facher Vergrößerung und
- Fig. 6b:: eine Abbildung nach Fig. 6a, jedoch in 200-facher Vergrößerung.

### Vergleichsbeispiel 1 :

Das für die Zwecke dieses Versuchsbeispiels verwendete organische Polymer ist ein Kunststoff-Hartschaum aus Polystyrol in Form eines Polystyrol-Granulats. Dieses Polystyrol-Granulat wird mit dem Rapsöl in der Weise belegt, daß auf 100 g des Granulats etwa 5 g Öl gerechnet werden, die im Fall dieses Ausführungsbeispiels durch Zerstäuben des Öls auf dem Granulat verteilt werden. Bei dem Polystyrol-Granulat handelt es sich um ein handelsüblich von der BASF AG, Deutschland, erhältliches Granulat.

Nach der Belegung des Polystyrol-Granulats mit dem Rapsöl fühlt sich das Granulat bei stärkerem Druck leicht klebrig an, ohne jedoch besonders zusammenzuklumpen.

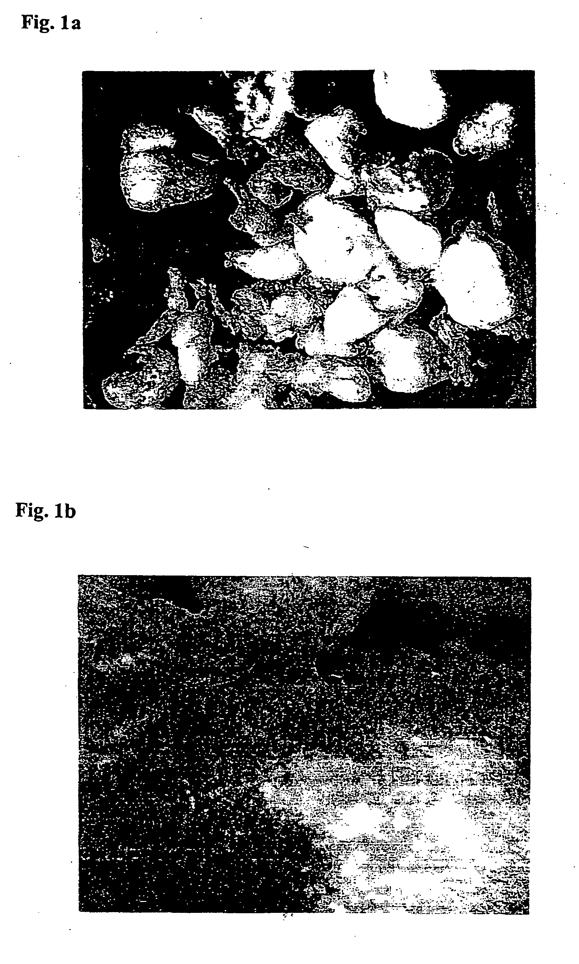

Das auf diese Weise mit dem Rapsöl belegte Polystyrol-Granulat ist mit einem Stereo-Lichtmikroskop vom Typ Olympus SZX 9 in zehnfacher Vergrößerung aufgenommen worden und hier als Fig. 1a dargestellt. Mit der weiteren Fig. 1b ist ein Ausschnitt desselben mit dem Rapsöl belegten Granulats, jedoch in 57-facher Vergrößerung dargestellt. Die Rapsöl-Belegung ist hier gut zu erkennen.

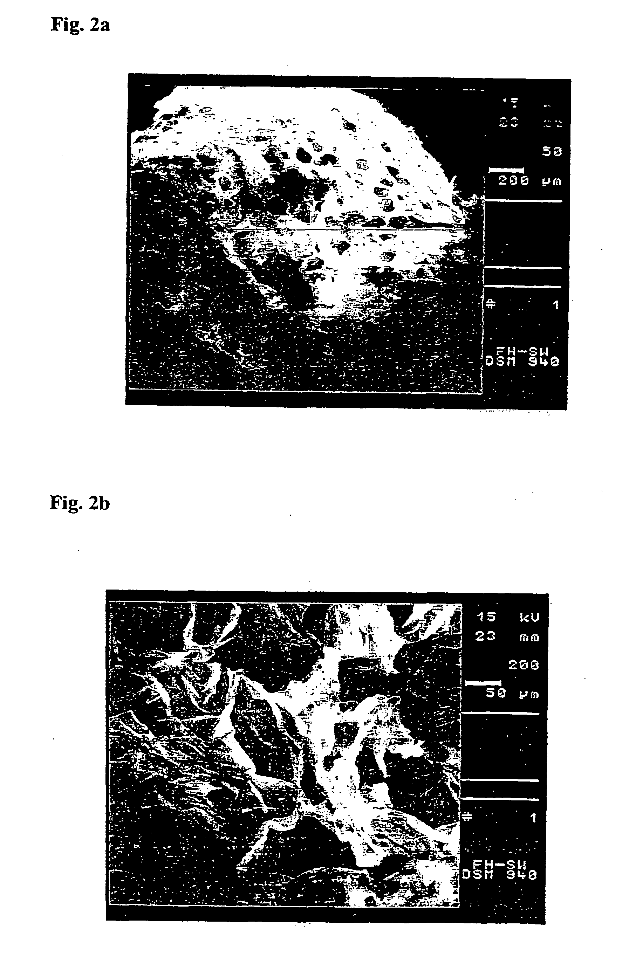

Die gleichen Aufnahmen werden zusätzlich mit einem digitalen Rasterelektronenmikroskop von Typ DSM 940 von Zeiss durchgeführt. Die Ergebnisse sind in Fig. 2a mit 50-facher Vergrößerung und in Fig. 2b mit 200-facher Vergrößerung dargestellt.

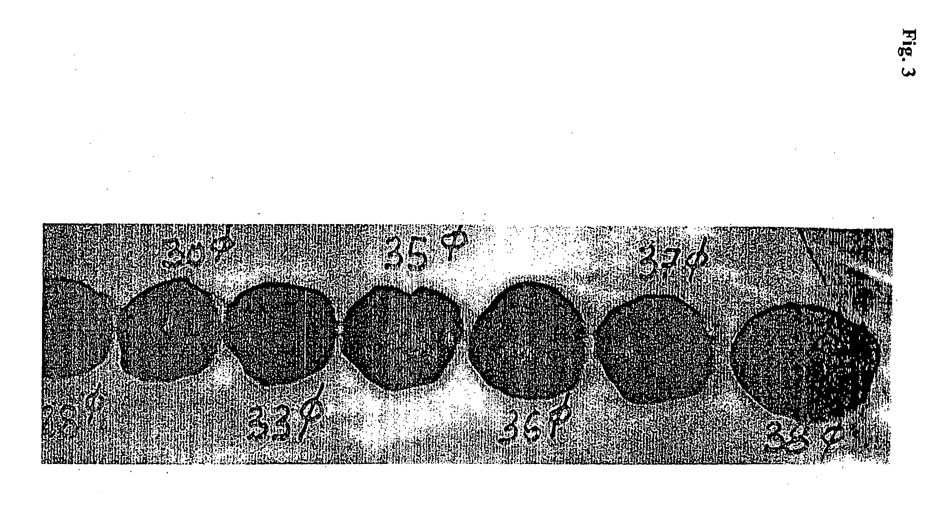

In einer einfachen Versuchsanordnung, bestehend aus einem mit Wasser gefüllten. Glaszylinder mit einem Durchmesser von 40 cfn und einer Höhe von 50 cm, wurde Erdöl als dünne Schicht auf die Wasseroberfläche in der Weise aufgebracht, daß sich ein zusammenhängender Ölteppich ergab. Anschließend wurde zerspanter Polystyrol-Schaum, der mit einem Rapsöl fein belegt war, auf diese Oberfläche geschüttet. Innerhalb kurzer Zeit wurde das auf der Wasseroberfläche befindliche Erdöl von dem mit dem Rapsöl belegten Polystyrol- Schaum schubweise aufgesogen, ohne daß die Oberfläche in irgendeiner Form gerührt oder berührt worden ist. Selbst bewußt auf der Oberfläche angehäufte Mengen des zerkleinerten Polystyrol-Schaums wurden von dem Erdöl erreicht. Es war erstaunlich zu beobachten, daß das Erdöl sogar auf die Haufen heraufkletterte. Es war quasi eine Sogwirkung zu beobachten. Bei entsprechend ausgewählten Mengenverhältnissen entstanden im wesentlichen regelmäßig geformte kugelförmige Gebilde, die aufgrund des Erdöls schwarz aussahen. Die so erhaltenen kugelförmigen Gebilde aus dem das Erdöl aufnehmenden Polystyrol-Schaum sind in Fig. 3 näher dargestellt.

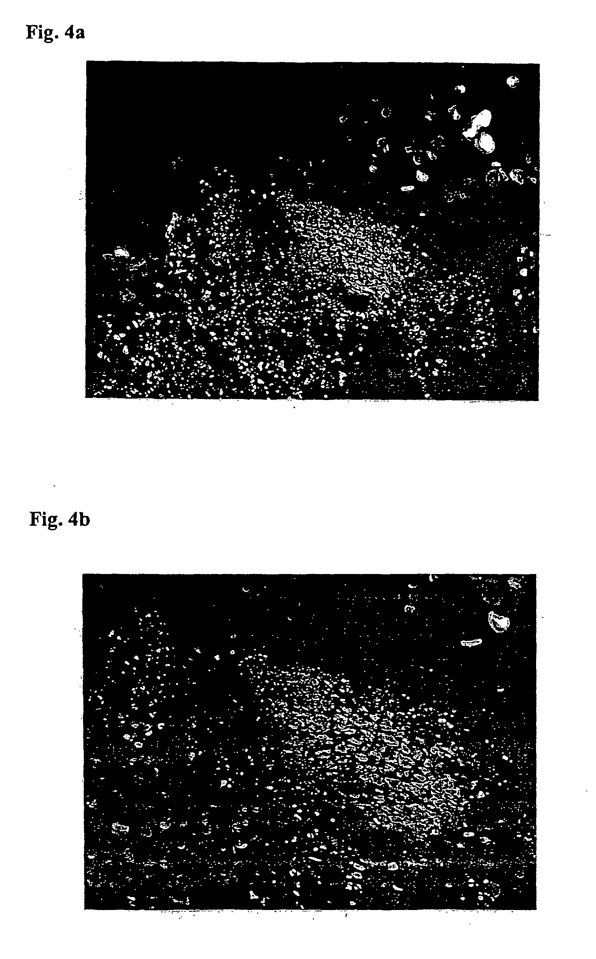

Zur weiteren Untersuchung wurde ein so erhaltenes kugelförmiges Gebilde aus dem Polystyrol-Hartschaum, welche das Erdöl aufgenommen hat, mit einer Rasierklinge durchtrennt und der Querschnitt mit einem Stereo-Lichtmikroskop vom Typ Olympus SZX 9 aufgenommen. In der lichtmikroskopischen Aufnahme ist die feine Äderung des Polystyrol-Granulats, welches das Erdöl aufgenommen hat, sowie die sich um ein mittig durchtrenntes Korn drängende Erdölmasse deutlich zu erkennen. Die Aufnahme zeigt eine 32-fache Vergrößerung und ist hier als Fig. 4a dargestellt.

Den gleichen Querschnitt durch ein solches kugelförmiges Gebilde als lichtmikroskopische Aufnahme wieder mit dem Stereo-Lichtmikroskop vom Typ Olympus SZX 9, jedoch in 57-facher Vergrößerung ist als Fig. 4b abgebildet und zeigt die in Fig. 4a dargestellten Details noch einmal etwas deutlicher.

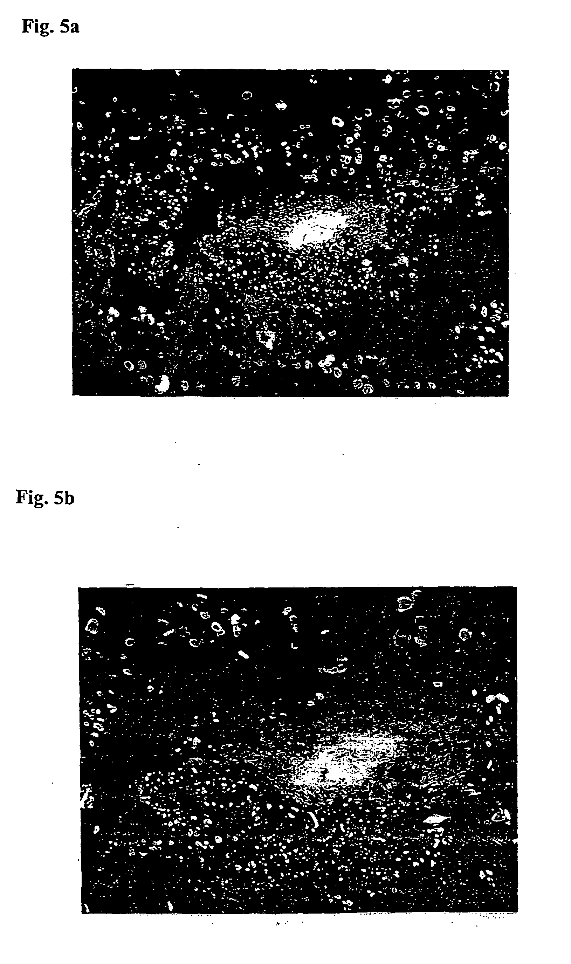

Das durch die Aufnahme des Erdöls von dem mit dem Rapsöl belegten Polystyrol-Granulat resultierende kugelförmige Gebilde wurde an einer weiteren Stelle ebenfalls mittels einer Rasierklinge nochmals durchtrennt und zwar in der Weise, daß ein nahe der äußerlich sichtbaren Begrenzung befindliches Granulatkorn angeschnitten wurde. Die daraus resultierende, mit dem gleichen Lichtmikroskop wie zuvor durchgeführte Aufnahme in 32-facher Vergrößerung zeigt Fig. 5a. In dieser Abbildung ist der Drang des Erdöls und seine Ausrichtung auf dem Granulatkorn vergleichbar mit der Wirkung eines Magneten auf Eisenfeilspäne deutlich sichtbar.

Dieselbe Aufnahme, jedoch in 57-facher Vergrößerung, ist in Fig. 5b dargestellt und zeigt das Phänomen der Ausrichtung des Erdöls in Richtung auf das Granulatkom noch einmal deutlicher.

Die gleichen Aufnahmen wurden zusätzlich mit einem digitalen Rasterelektronenmikroskop vom Typ DSM 940 von Zeiss durchgeführt. Sie dienen dazu, nähere Auskünfte über die Oberflächenstruktur des sich auf dem mit Rapsöl belegten Polystyrolgranulatkoms anordnenden Erdöl zu geben und gleichzeitig nähere Aufschlüsse über die Verbindung zwischen dem Polystyrol-Granulatkom und dem Erdöl zu geben.

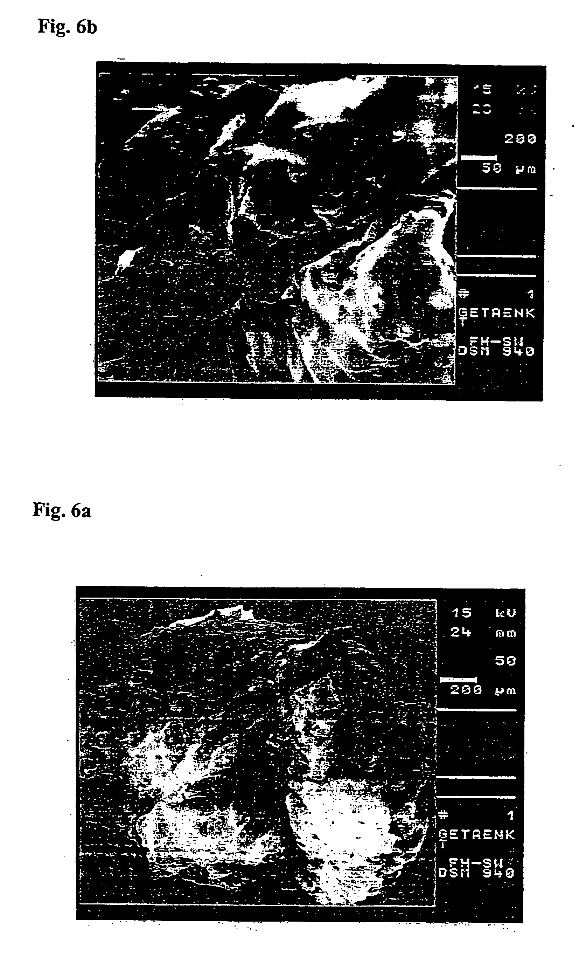

Eine erste rasterelektronenmikroskopische Aufnahme des Querschnitts durch das kugelförmige Gebilde zeigt in Fig. 6a ein vollkommen von dem Erdöl umschlossenes Granulat-Korn in 50-facher Vergrößerung. Dabei wird der Schichtaufbau des Erdöls deutlich sichtbar. Da die Arbeitsspannung des verwendeten Elektronenmikroskops 15.000 Volt betrug, sind die Elektronenstrahlen des Mikroskops in der Lage, das Erdöl zu durchdringen, so daß sie es nicht schwarz, sondern weiß erscheinen lassen.

Dieselbe Aufnahme, jedoch mit 200-facher Vergrößerung ist in Fig. 6b dargestellt. Der dadurch sichtbare Ausschnitt von der Oberfläche des auf dem Granulat-Korn gebundenen Erdöl ist dadurch zwar kleiner, zeigt aber die Erdöl-Schichtung nochmals deutlicher.

Mit diesen rasterelektronenmikroskopischen Aufnahmen ist nicht nur ein näherer Aufschluß über die schon vermutete innige Verbindung zwischen dem Erdöl und dem mit dem Rapsöl belegten Polystyrol-Granulat anschaulich dargestellt worden, sondern es läßt sich auch über die Natur der Verbindung zwischen dem Granulat und dem Erdöl ein Rückschluß ziehen. Bekanntlich sind rasterelektronenmikroskopische Aufnahmen nur durch Anlegen eines Vakuums, in das die zu untersuchende Probe eingebracht wird, möglich. Das hier verwendete Mikroskop vom Typ DSM 940, das in einem Vergrößerungsbereich von 5 bis 100.000 arbeitet, verwendete ein Hochvakuum von 10⁻⁴ Torr bei der Aufnahme der Proben. Wenn die Verbindung zwischen dem Granulat und dem davon angezogenen und daher darauf angelagertem Erdöl nur physikalischer Natur wäre, hätte diese Verbindung unter Einwirkung des Hochvakuums keinen Bestand haben können und als Folge davon hätte sich das Erdöl von der Oberfläche des Granulats lösen und das hochempfindliche Rasterelektronenmikroskop verschmutzen müssen. Aufgrund der Tatsache, daß die durch die Aufnahme des Erdöls gebildeten kugelförmigen Gebilde aus dem Polystyrol-Hartschaum bzw. der Teil davon, der zur Untersuchung des Querschnitts in das Hochvakuum des Rasterelektronenmikroskop befördert wurde, keine Spur des angelagerten Erdöls freigegeben hat, wird deutlich, daß die Verbindung zwischen dem Erdöl und dem Polystyrol-Granulat keinesfalls physikalischer, sondern chemischer Natur sein muß. Es handelt sich somit um eine durch Adsorption hervorgerufene echte chemische Verbindung der beiden Komponenten.

In vielfältigen Versuchen wurde festgestellt, daß sich aufgrund dieser Adsorptionsverbindung zwischen dem Polystyrol-Hartschaum bzw. Polystyrol-Granulat mit dem Öl und damit mit dem Aufbringen des Öls auf das Kapillarsystem des in zerspanter oder grundsätzlich verkleinerten Form vorliegenden Polystyrols die ihm ohnehin schon innewohnende Fähigkeit zum Ölbinden verstärkt und vor allem die Feuchtigkeits- und Wasseraufnahme verhindert wird. Die festgestellte leichte Klebrigkeit läßt schon auf die Fähigkeit dieses neuen Produkts als Bindemittel schließen. Die Anwendungsmöglichkeiten sind jedoch sehr vielfältig und sollen mit den folgenden Beispielen exemplarisch dargestellt werden. Dabei ist zu beachten, daß bei diesen Einsatzgebieten zweckmäßigerweise nicht nur das pflanzliche Öl als solches, sondern in Form seiner Bestandteile bzw. Derivate verwendet wird. Bei den nachfolgend in den Beispielen eingesetzten Bestandteilen und Derivaten handelt es sich um in der Regel handelsüblich erhältliche Produkte oder ihre Herstellung ist im Stand der Technik hinlänglich bekannt.

### Beispiel 2: Herstellung eines Klebstoffs

### Beispiel 2a:

90 Gew.-Teile eines handelsüblich als Acronal 290 D von der BASF AG, Deutschland erhältlichen wäßrigen Styrol/Butylacrylat-Dispersion wurden mit 10 Gew.-Teilen Rapsöl unter Temperaturerhöhung zu einer homogenen Dispersion verrührt, auf verschiedenen Oberflächen, wie Holz, Kunststoff und Pappe aufgetragen und antrocknen gelassen. Die so erhaltenen Oberflächen wurden mit Papier abgedeckt, das sich nicht mehr ohne Zerreißen abziehen ließ.

### Beispiel 2b:

85 Gew.-Teile eines handelsüblich als Acronal 290 D von der BASF AG, Deutschland erhältlichen wäßrigen Styrol/Butylacrylat-Dispersion wurden mit 15 Gew.-Teilen RME unter Temperaturerhöhung zu einer homogenen Dispersion verrührt, auf verschiedenen Oberflächen, wie Holz, Kunststoff und Pappe aufgetragen und antrocknen gelassen. Die so erhaltenen Oberflächen wurden mit Papier abgedeckt, das sich ebenfalls nicht mehr ohne Zerreißen abziehen ließ.

### Beispiel 3: Herstellung eines Zusatzes für hydraulische Bindemittel

Die weiter oben beschriebenen Klebstoffzusammensetzungen dienen ebenfalls als Basis für die Vergütung von Baustoffen, wobei hier wieder Acronal 290 D verwendet und für folgende Einsatzzwecke getestet wurde:

### a) Zusatz in Mörtel-Schlämmen:

Die Verwendung der erfindungsgemäßen Zusammensetzung als Vergütung in den Mörtel-Schlämmen verbesserte die Plastizität und Verarbeitbarkeit des Mörtels, ohne den Wasseranteil zu erhöhen.

Außerdem wurde eine verbesserte Festigkeit, Zähigkeit, Haftung und ein verbessertes Spannungsverhalten festgestellt.

### b) Zusatz in Zementmörtel und Betonspachtelmassen:

Hier war eine erhöhte Festigkeit und Elastizität und ein besserer Haftverbund zu verzeichnen. Die Rißbildung und der Abrieb wurden vermindert. Besonders ist hervorzuheben, daß in Langzeitversuchen eine Verbesserung des Korrosionsschutzes festzustellen war.

### c) Zusatz für Estrich- und Verlegemörtel:

Im Vergleich zu herkömmlichen Produkten konnten dünnere Verbund-EstrichSchichten verwendet werden. Die Abriebfestigkeit und Elastizität wurde erhöht und die Haftung des Mörtels und/oder der Putzschichten auf glattem Untergrund verbessert. Auch der Haftverbund von Verlegemörteln für Fliesen und Platten aus Naturstein war deutlich höher als bei herkömmlichen Produkten.

### d) Ausbesserungsarbeiten

Die erfindungsgemäßen Zusammensetzungen konnten ohne Schwierigkeiten auch für Reparaturen an schon bestehenden Betonteilen, einschließlich Betonfertigteilen, Estrichen und an Putz verwendet werden.

## Patentansprüche

1. Verfahren zur Herstellung eines modifizierten Bindemittels oder Gerüststoffs auf der Basis eines Kunststoffs und mindestens einem Pflanzenöl, Pflanzenölbestandteil oder Pflanzenölderivat wobei, der Kunststoff Polystyrol oder ein modifiziertes Polystyrol ist und, in zerkleinerter Form oder in Form eine Homo-oder eines Copolymerates vorliegt
**dadurch gekennzeichnet dass** die Herstellung des modifizierten Bindemittels oder Gerüststoffs folgende Schritte umfasst:
• Reaktion des Kunststoffs in zerspanter oder feinverteilter Form mit einem ersten Pflanzenöl, Pflanzenölbestandteil oder Pflanzenölderivat,
• Reaktion des Zwischenproduktes aus dem ersten Reaktionsschritt mit demselben oder einem weiteren Pflanzenöl, Pflanzenölbestandteil oder Pflanzenölderivat unter Bildung des Endproduktes
wobei es sich bei dem modifizierten Bindemittel oder Gerüststoff um eine Adsorptionsverbindung aus dem Polystyrol oder dem modifizierten Polystyrol und dem Pflanzenöl, Pflanzenölbestandteil oder Pflanzenölderivat handelt.

2. Verfahren nach Anspruch 1, wobei es sich bei dem modifizierten Polystyrol um alpha-Methylstyrol handelt.

3. Verfahren nach Anspruch 1, wobei es sich bei dem Copolymerisat des Polystyrols um Styrol/(Meth)acrylat-Copolymer handelt.

4. Verfahren nach Anspruch 1, wobei das Pflanzenöl ausgewählt ist aus der Gruppe bestehend aus: Rapsöl, Sonnenblumenkernöl, Sojaöl, Rizinusöl, Olivenöl, Leinöl, Kokosöl, Palmöl oder Mischungen davon.

5. Verfahren nach Anspruch 1, wobei der Pflanzenölbestandteil ausgewählt ist aus der Gruppe bestehend aus Mono-, Di- oder Triestern des Glycerins und höheren geradzahligen Fettsäuren mit einer Kettenlänge C₄ bis C₂₄.

6. Verfahren nach Anspruch 1, wobei das Pflanzenölderivat ein Ester ist.

7. Verfahren nach den Ansprüchen 1 und 6, wobei der Ester ein Methylester einer mehrfach ungesättigten Fettsäure ist.

8. Verfahren nach den Ansprüchen 1, 6 und 7, wobei der Methylester ausgewählt ist aus der Gruppe bestehend aus: Rapsfettsäuremethylester, Rizinolsäuremethylester, Ölsäuremethylester, Linolsäuremethylester, Laurinsäuremethylester.

9. Verwendung eines modifizierten Bindemittels oder Gerüststoffes hergestellt gemäß einem Verfahrens nach mindestens einem der Ansprüche 1 bis 8 zur Anwendung in Baustoffen.

10. Verwendung nach Anspruch 9, wobei das modifizierte Bindemittel oder der modifizierte Gerüststoff ein Zusatz in Baustoffen ist.

11. Verwendung nach mindestens einem der Ansprüche 9 und 10, wobei es sich bei dem Baustoff um Klebstoff oder Dichtungsmasse handelt.

12. Verwendung nach mindestens einem der Ansprüche 9 und 10, wobei es sich bei dem Baustoff um hydraulische Bindemittel, Gips, nichthydraulischen Kalk und Trockenmörtelprodukte handelt.

## Claims

1. Method of manufacturing a modified binder or building material on the basis of a synthetic material with at least one vegetable oil, vegetable oil constituent or vegetable oil derivate, wherein the synthetic material is polystyrene or a modified polystyrene and is present in a fragmented form or in the form of a monopolymerisate or copolymerisate
**characterized in that** the manufacturing of the modified binder or building material comprises the following steps:
• reacting the synthetic material in a machined or finely dispersed form with a first vegetable oil, vegetable oil constituent or vegetable oil derivate,
• reacting the intermediate product from the first reaction step with an identical or another vegetable oil, vegetable oil constituent or vegetable oil derivate to form the final product
wherein the modified binder or building material represents an absorption compound from the polystyrene or the modified polystyrene and the vegetable oil, vegetable oil constituent or vegetable oil derivate.

2. Method according to claim 1, wherein the modified polystyrene is α-methyl styrene.

3. Method according to claim 1, wherein the copolymerisate of polystyrene is a styrene/(meth-)acrylate copolymer.

4. Method according to claim 1, wherein the vegetable oil is selected from the group consisting of rape-seed oil, sunflower seed-oil, soybean oil, castor oil, olive oil, linseed oil, coconut oil, palm oil or mixtures thereof.

5. Method according to claim 1, wherein the vegetable oil constituent is selected from the group consisting of mono-, di- or triesters of glycerin and higher even-numbered fatty acids with chain lengths ranging from C₄ to C₂₄.

6. Method according to claim 1, wherein the vegetable oil derivative is an ester.

7. Method according to claim 1 and 6, wherein the ester is a methyl ester of a multiple-unsaturated fatty acid.

8. Method according to claim 1, 6 and 7, wherein the methyl ester is selected from the group consisting of rape-seed fatty acid methyl ester, ricinoleic acid methyl ester, oleic acid methyl ester, linoleic acid methyl ester and lauric acid methyl ester.

9. Use of a modified binder or building material produced by a method according to at least one of claims 1 to 8 for use in construction materials.

10. Use according to claim 9, wherein the modified binder or the modified building material is an additive to construction materials.

11. Use according to at least one of claims 9 and 10, wherein the construction material is an adhesive or sealant.

12. Use according to at least one of claims 9 and 10, wherein the construction material is a hydraulic binding agent, gypsum, a non-hydraulic lime and a dry mortar product.

## Revendications

1. Procédé pour la préparation d'un liant ou d'un matériau de soutien modifié à base d'une matière plastique et d'au moins une huile végétale, d'un constituant d'huile végétale ou d'un dérivé d'huile végétale dans lequel, la matière plastique est le polystyrène ou un polystyrène modifié et, est présente dans une forme broyée ou dans une forme d'un homo- ou d'un copolymère
**caractérisé en ce que** la préparation du liant ou du matériau de soutien modifié comprend les étapes suivantes :
• de réaction de la matière plastique dans une forme de copeaux ou finement distribuée avec une première huile végétale, un premier constituant d'huile végétale ou un premier dérivé d'huile végétale,
• de réaction du produit intermédiaire de la première étape de réaction avec la même huile végétale ou une autre, le même constituant d'huile végétale ou un autre ou le même dérivé d'huile végétale ou un autre avec la formation d'un produit final
dans lequel il s'agit pour le liant ou le matériau de soutien modifié d'un composé d'adsorption constitué du polystyrène ou du polystyrène modifié et de l'huile végétale, du constituant d'huile végétale ou du dérivé d'huile végétale.

2. Procédé selon la revendication 1, dans lequel il s'agit pour le polystyrène modifié d'alpha-méthylstyrène.

3. Procédé selon la revendication 1, dans lequel il s'agit pour le copolymère du polystyrène d'un copolymère de styrène/(méth)acrylate.

4. Procédé selon la revendication 1, dans lequel l'huile végétale est choisie dans le groupe constitué de : huile de colza, huile de tournesol, huile de soja, huile de ricin, huile d'olive, huile de lin, huile de coco, huile de palme ou mélanges de celles-ci.

5. Procédé selon la revendication 1, dans lequel le constituant d'huile végétale est choisi dans le groupe constitué de mono-, di- ou triesters de la glycérine et d'acides gras linéaires supérieurs avec une longueur de chaîne de C₄ à C₂₄.

6. Procédé selon la revendication 1, dans lequel le dérivé d'huile végétale est un ester.

7. Procédé selon les revendications 1 et 6, dans lequel l'ester est un ester méthylique d'un acide gras plusieurs fois insaturé.

8. Procédé selon les revendications 1, 6 et 7, dans lequel l'ester méthylique est choisi dans le groupe constitué de : ester méthylique d'acide gras de colza, ester méthylique d'acide ricinoléique, ester méthylique d'acide oléique, ester méthylique d'acide linoléique, ester méthylique d'acide laurique.

9. Utilisation d'un liant ou d'un matériau de soutien modifié préparé selon un procédé selon au moins l'une des revendications 1 à 8 pour une application dans des matériaux de construction.

10. Utilisation selon la revendication 9, dans laquelle le liant modifié ou le matériau de soutien modifié est un additif dans des matériaux de construction.

11. Utilisation selon au moins l'une des revendications 9 et 10, dans laquelle il s'agit pour le matériau de construction d'un adhésif ou d'une masse d'étanchéité.

12. Utilisation selon au moins l'une des revendications 9 et 10, dans laquelle il s'agit pour le matériau de construction de liant hydraulique, de gypse, de chaux non hydraulique et de produits de mortier sec.
